# EUROPEAN PATENT APPLICATION

(11) **EP 4 119 581 A1**
(43) Date of publication of application: **18.01.2023**
(21) Application number: 21185562.2
(22) Date of filing: 14.07.2021
(51) Int. Cl.: C07K 16/30

(54) **NOVEL FAB DIMERS**

(71) Applicant: Helmholtz Zentrum München Deutsches Forschungszentrum für Gesundheit und Umwelt GmbH, 85764 Neuherberg (DE)
(72) Inventor: ZEIDLER, Reinhard, 82140 Olching (DE)
(74) Representative: Schiweck Weinzierl Koch Patentanwälte Partnerschaft mbB

(57) **Abstract**

The present invention relates to a novel dimer composed of a first Fab monomer and a second Fab monomer, each Fab monomer comprising a VH and a VL region, wherein two of such VH or VL regions are covalently linked by a disulfide bond between an additional non-naturally occurring cysteine residue at their respective N-termini.

## Description

The present invention relates to a novel dimer composed of a first Fab monomer and a second Fab monomer, each Fab monomer comprising a VH and a VL region, wherein two of such VH or VL regions are covalently linked by a disulfide bond between an additional non-naturally occurring cysteine residue at their respective N-termini.

Carbonic anhydrases are a family of enzymes that catalyze the reversible hydration of carbonic acid to bicarbonate and protons, and thus participate in the maintenance of pH homeostasis in the body (Badger et al., Annu Rev Plant Physiol Plant Mol Bio (1994), 45: 369-392). In the absence of a catalyst this reaction occurs rather slowly. Since most carbonic anhydrases contain a zinc ion in their active site they are classified as being metalloenzymes. The family of carbonic anhydrases has several members. There are at least five distinct carbonic anhydrase subfamilies (α, β, γ, δ and ε). These subfamilies have no significant amino acid sequence similarity and in most cases are thought to be an example of convergent evolution. The α-carbonic anhydrases (CAs) are found in mammals. The members of this subfamily can be distinguished with respect to their kinetics, tissue expression and subcellular localization (Kivela et. al., World J Gastroenterol (2005), 11(2): 155-163).

α-CA enzymes are divided into four broad subgroups, namely into the cytosolic CAs (CA-I, CA-II, CA-III, CA-VII and CA-XIII), mitochondrial CAs (CA-VA and CA-VB), secreted CAs (CAVI), and membrane-associated CAs (CA-IV, CA-IX, CA-XII, CA-XIV and CA-XV) (Breton et al. (2001), JOP 2 (4 Suppl): 159-64). Moreover, there are three "catalytic" CA isoforms (CA-VIII, CA-X, and CA-XI) whose functions remain unclear. Of all these CAs several isoforms exist in addition.

CA-II, CA-IX and CA-XII have been associated with neoplastic processes, and they are potential histological and prognostic biomarkers of various tumors (Nordfors et al. (2010), BMC cancer; 10:148). CA-II is the most widely expressed member of the α-CA gene family, being present in virtually every human tissue and organ. It is catalytically one of the most efficient enzymes known. It is present to some extent in malignant cells, and, interestingly, it has been recently shown to be ectopically expressed in the endothelial cells of tumor neovessels. The transmembrane enzyme, CA-IX, was first recognized as a novel tumor-associated antigen expressed in several types of human carcinomas as well as in normal gastrointestinal tissue. CA-IX has been functionally linked to cell adhesion, differentiation, proliferation and oncogenic processes, and its enzymatic activity is comparable to CA II. Another transmembrane CA isozyme, CA-XII, was first found in normal kidney tissue and renal cell carcinoma. Further studies have shown that it is expressed in several other tumors (Ulmasov et al., PNAS (2000), 97(26): 1412-1417), but also in some normal organs such as the colon and uterus. The X-ray crystallographic structure of human CA-XII reveals that it is a bitopic dimeric protein whose short intracellular C-terminus is placed on the opposite side of the active site domains such that the latter face is towards the extracellular space.

High expression of CA-II, CA-IX and CA-XII in tumors, particularly under hypoxic conditions has further suggested that these enzymes may functionally participate in the invasion process, which is facilitated by acidification of the extracellular space. In favor of this hypothesis, it has been shown in vitro that CA inhibitors can reduce the invasion capacity and proliferation of cancer cells (Manokaran et al. (2008), J Biomed Nanotechnol., 4(4):491-498). In particular, CA IX and XII seem to be regulated by similar mechanisms, as transcription of these isozymes is induced in tumors under hypoxic conditions through hypoxia inducible factor-1 alpha (HIF-1α)-mediated pathways (Chiche et al. (2009)). In addition, it has been shown that the expression of CA-XII is highly correlated with estrogen receptor alpha (ERα) in breast tumors (Barnett et al. (2008), Cancer Res 68:3505-3515). To explain the importance of CAs in cancer progression in more detail, the rapidly proliferating tumor cells quickly overgrow such that the diffusion of oxygen from the nearest blood vessel (100-150 µm) is impaired. Consequently, tumor cells receive low level of oxygen, causing local hypoxic centers and tissue necrosis. Hypoxia creates selective pressure in cells to adopt to stress conditions, resulting in the expression of approximately 50 additional proteins, including the enzymes involved in pH homeostasis (Potter et al. (2004), Gell Cycle, 3:164-167). As explained above, the latter is accomplished, at least in part, by an intricate coordination between selected carbonic anhydrase (CA) isozymes, particularly CA-II, CA-IX and CA-XII. A direct link between CA XII and cancer has been demonstrated by Proescholdt et al. (2005), Neuro Onco 7:465-4 75. Here it is demonstrated that CA XII expression is upregulated in intrinsic and metastatic brain tumors as compared to normal brain tissue. Furthermore, Ilie et al. (2011), In J Cancer, 128(7): 1614-23 and Hynninen et al. (2006), Histopathology, 49:594-602 showed overexpression of CA XII in tissues from resectable non-small lung cancer and ovarian cancer, respectively. Hsieh et al. (2010), Eur J Gell Biol, 89:598-606 revealed in vivo and in vitro that CA XII is associated with invasion and metastasis of tumor cell lines.

Moreover, CA-inhibitors, in particular inhibitors of CA-II and CA-XII are used to reduce intraocular pressure and thus to treat ocular hypertension (Al-Barrag et al. (2009), Clinical Ophthalomology 3:357-362). Also, CA-inhibitors were shown to be useful in the treatment of glaucoma (Haapasalo et al. (2008), Neuro Oncology 3:357-362, and Vullo et al. 2005).

Accordingly, CAs, and in particular CA-XII, are known to play a role in hypoxia, cancer and eye diseases and are therefore important targets for a therapeutical treatment or diagnosis (Thiry et al 2008, Vulo et al 2005, and Haapasalo et al. (2008), Neuro Oncology 3:357-362). Although systemic carbonic anhydrase inhibitors are known in the art, they are associated with adverse side effects, such as acid-base disturbance, hypersensitivity reactions, and fatal aplastic anemia (Gross et al. (1988), Am J Opthamol., Naeser et al. (1986), Acta Opthalmol., 64:330-337, Mastropasqua et al. (1998), 212:318-321; and Passp et al, Br J Opthalmol. 1985; 69:572-575). Thus, further means and methods are required that may be employed in the development of further therapeutic and diagnostic means for the above mentioned diseases.

These and further disadvantages need to be overcome. The present invention therefore addresses these needs and technical objectives and provides a solution as described herein and as defined in the claims.

The present invention relates to a dimer composed of a first Fab monomer and a second Fab monomer, each Fab monomer comprising a VH and a VL region, wherein
(i) the VL region of the first Fab monomer and the VL region of the second Fab monomer are covalently linked by a disulfide bond between an additional non-naturally occurring cysteine residue at the N-terminus of the VL region of the first Fab monomer and the N-terminus of the VL region of the second Fab monomer;
(ii) the VH region of the first Fab monomer and the VH region of the second Fab monomer are covalently linked by a disulfide bond between an additional non-naturally occurring cysteine residue at the N-terminus of the VH region of the first Fab monomer and the N-terminus of the VH region of the second Fab monomer;
(iii) the VL region of the first Fab monomer and the VH region of the second Fab monomer are covalently linked by a disulfide bond between an additional non-naturally occurring cysteine residue at the N-terminus of the VL region of the first Fab monomer and the N-terminus of the VH region of the second Fab monomer; or
(iv) the VH region of the first Fab monomer and the VL region of the second Fab monomer are covalently linked by a disulfide bond between an additional non-naturally occurring cysteine residue at the N-terminus of the VH region of the first Fab monomer and the N-terminus of the VL region of the second Fab monomer.

As has been found in context with the present invention, Fab monomers were dimerized via a cysteine-bond (disulfide bond) between an additional non-naturally occurring cysteine residue at the N-terminus of a VL and/or VH region of a Fab monomer. Accordingly, in accordance with the present invention, the presence of an additional non-naturally occurring cysteine residue at the N-terminus of the VL region and/or VH region of a Fab monomer allows building of a cysteine-bond (disulfide bond) between the Fab monomers to build a Fab dimer as described and provided herein. As was surprisingly found in context with the present invention, and as shown herein, these Fab dimers comprising a cysteine-bond (disulfide bond) exhibit increased binding affinity and/or avidity to target structures (e.g., antigens) in acidic environments compared to the respective Fab monomers (directed to the same target structures (e.g., antigens)) not comprising such cysteine-bond (disulfide bond), or not comprising an additional non-naturally occurring cysteine residue at the N-terminus of the VL and/or VH region. In accordance with the present invention, this increased binding affinity and/or avidity to target structures (e.g., antigens) in acidic environments may be particularly important in cases where the target structures (also referred to herein as targets; e.g., antigens) which are bound by the Fab dimers are present in or relevant for treating or diagnosing cancer because of the acidic microenvironment in tumors (see, e.g., Estrella et al., Microenviron Immunol (2013), 73(5): 1524-1535; Boedtkjer et al., Annual Rev Physiol (2020), 82: 103-126; Pillai et al., Cancer Metastasis Rev (2019), 38: 205-222; Ji et al., Cancer Metastasis Rev (2019): 38: 103-112; Gatenby et al., Nature Rev Cancer (2004): 891-899).

Generally, in context with the present invention, the additional non-naturally occurring cysteine residue at the N-terminus of the VL and/or VH region of a Fab monomer as described herein may be at any position within the respective VL or VH region which is more N- than C-terminal, In one embodiment of the present invention, it may be within the first 10 N-terminal amino acids of the respective VL or VH region (the first amino acid of the respective VL or VH region being counted as position 1 at the N-terminus), more preferably within the first 3 N-terminal amino acids, most preferably within the first 2 N-terminal amino acids. That is, on this embodiment of the present invention, the non-naturally occurring cysteine residue at the N-terminus of the VL region or VH region is at amino acid position 1 or amino acid position 2, wherein amino acids are counted from the N-terminus of the VL region or VH region, respectively, with position 1 being the first amino acid at the N-terminus.

As used herein, and as readily understood by those of skill in the art, a "Fab" (or "Fab molecule") is a fragment of an antibody, e.g. as described herein, and is commonly known in the art. It may comprise a binding agent comprising a variable light (VL) chain or region and a variable heavy (VH) chain or region, which together form a binding region or motif which allows binding of the Fab to a target structure, e.g., an antigen or epitope. The VH chain or region and the VL chain or region of a "Fab" may further comprise a constant region or parts of a constant region. As used herein, where reasonably applicable for those of skill in the art, the term "Fab" may also comprise F(ab')₂, Fab', Fv, scFv, Fd, dAb, and other antibody fragments that retain antigen-binding function. Typically, such fragments would comprise an antigen-binding domain and have the same properties as the Fab described herein.

In context with the present invention, as used herein, "additional" in context with "additional non-naturally occurring cysteine residue" at the N-terminus of the VL and/or VH region of a Fab monomer as described herein may mean (i) that the cysteine residue is present in the respective N-terminus of the VL and/or VH region of a Fab monomer in addition to the other amino acids comprised by the respective reference (i.e. natural) sequence of the respective VL and/or VH region (thus expanding the total amino acid sequence length of the reference VL or VH region by 1 amino acid), or (ii) that it substitutes one of the amino acids comprised by the respective reference (i.e. natural) sequence of the respective VL and/or VH region (thus not changing the total amino acid sequence of the reference VL or VH region, but adding 1 cysteine residue compared to said reference sequence). In a preferred embodiment of the present invention, "additional" means an addition of a cysteine residue to the other amino acids comprised by the respective reference (i.e. natural) sequence of the respective VL and/or VH region according to (i) of the previous sentence.

Likewise, in context with the present invention, as used herein, "non-natural occurring" in context with "additional non-naturally occurring cysteine residue" at the N-terminus of the VL and/or VH region of a Fab monomer as described herein may mean that the additional cysteine residue does not exist in the amino acid sequence of the respective reference VL or VH region, either at this respective position or at all, or neither at this respective position or at all.

In accordance with the present invention, said first Fab monomer and said second Fab monomer whose VL and/or VH regions are covalently linked by a disulfide bond between an additional non-naturally occurring cysteine residue at the N-terminus of their respective VL and/or VH regions may be the same or different. In both cases, said first Fab monomer and said second Fab monomer whose VL and/or VH regions are covalently linked by a disulfide bond between an additional non-naturally occurring cysteine residue at the N-terminus of their respective VL and/or VH regions may be directed against the same target or against different targets (e.g., antigens). In one embodiment, in accordance with the present invention, said first Fab monomer and said second Fab monomer whose VL and/or VH regions are covalently linked by a disulfide bond between an additional non-naturally occurring cysteine residue at the N-terminus of their respective VL and/or VH regions may be the same, and both Fab monomers are directed against the same target. In another embodiment of the present invention, said first Fab monomer and said second Fab monomer whose VL and/or VH regions are covalently linked by a disulfide bond between an additional non-naturally occurring cysteine residue at the N-terminus of their respective VL and/or VH regions may be different, and are directed against the same target. In yet another embodiment of the present invention, said first Fab monomer and said second Fab monomer whose VL and/or VH regions are covalently linked by a disulfide bond between an additional non-naturally occurring cysteine residue at the N-terminus of their respective VL and/or VH regions may be different, and are directed against different targets. In the latter embodiment of the present invention, said first Fab monomer is directed against a first target and said second Fab monomer is directed against a second target, said first and second target being different. In this case, the dimer as described and provided herein would be a bispecific Fab-dimer as it is directed against two different targets.

In accordance with the present invention, the target against which the Fab dimer of the present invention is directed may be any target which is desired to tag, mark, neutralize or otherwise bind to. In some embodiments of the present invention, such targets may be antigens present in subjects suffering from hypoxia or an eye disease, or in or on cancerous or tumorous cells, or otherwise be present in subjects suffering from cancer, particularly solid tumors. Such targets may be antigens or any other structure with peptidic, glycosidic and/or other portions, to which an antibody or fragments thereof (e.g., Fab or F(ab')2 fragments) may be able to bind to. In a specific embodiment of the present invention, the target against which the dimer of the present invention is directed is α-carbonic anhydrase XII (CA-XII).

Accordingly, in a specific embodiment of the present invention, the dimer as described and provided herein binds (preferably specifically binds) to CA-XII. In this context, in accordance with the present invention, the dimer as described and provided herein may bind (preferably specifically bind) to CA-XII with either one or both of the first and second Fab monomer as described herein, preferably both.

The term "specifically recognizing" (equally used herein with "specifically binding", "directed to" or "reacting with") means in accordance with this invention that the recognition molecule is capable of specifically interacting with and/or binding to at least two, preferably at least three, more preferably at least four amino acids of an epitope as defined herein. Such binding may be exemplified by the specificity of a "lock-and-key-principle". Thus, the term "specifically" in this context means that the recognition molecule binds to a given target epitope but does not essentially bind to another protein. The term "another protein" includes any protein including proteins closely related to or being homologous to the epitope against which the recognition molecule is directed to. However, the term "another protein" does not include that the recognition molecule cross-reacts with the epitope from a species different from that against which the recognition molecule was generated.

The term "does not essentially bind" as used herein means that the epitope recognition molecule of the present invention does not bind another protein, *i.e.* shows a cross-reactivity of less than 30%, preferably 20%, more preferably 10%, particularly preferably less than 9, 8, 7, 6 or 5% with another protein.

Specific binding is believed to be effected by specific motifs in the amino acid sequence of the binding domain and the antigen bind to each other as a result of their primary, secondary or tertiary structure as well as the result of secondary modifications of said structure. The specific interaction of the antigen-interaction-site with its specific antigen may result as well in a simple binding of said site to the antigen. Moreover, the specific interaction of the antigen-interaction-site with its specific antigen may alternatively result in the initiation of a signal, e.g. due to the induction of a change of the conformation of the antigen, an oligomerization of the antigen, etc. A preferred example of a binding domain in line with the present invention is an antibody. Typically, binding is considered "specific" when the binding affinity is higher than 10⁻⁶M. Preferably, binding is considered specific when binding affinity is about 10⁻¹¹ to 10⁻⁸ M (K_{D}), preferably of about 10⁻¹¹ to 10⁻⁹ M. If necessary, nonspecific binding can be reduced without substantially affecting specific binding by varying the binding conditions. Whether the recognition molecule specifically reacts as defined herein above can easily be tested, *inter alia,* by comparing the reaction of said recognition molecule with an epitope with the reaction of said recognition molecule with (an) other protein(s).

In one embodiment of the present invention, CA-XII is human CA-XII. In accordance with the present invention, it may furthermore be that the dimer as described and provided binds to at least one epitope of the extracellular domain CA-XII as described in WO2011/138279, or - preferably - in the region of the discontinuous, catalytic domain of CA-XII. The term "extracellular domain" according to the present invention is a term well-known in the art and also relates in conjunction with the present invention to the portion of the CA-XII extending into the extracellular environment. Also the term "catalytic domain" according to the present invention is a term well-known in the art and also relates in conjunction with the present invention to the portion of the CA-XII at which catalysis of carbonic acid to bicarbonate and protons occurs.

The terms "antigen "and "immunogen" are used interchangeably herein to refer to a molecule or substance which induces an immune response (preferably an antibody response) in an animal, preferably a non-human animal immunized therewith (i.e. the antigen is "immunogenic" in the animal), and which can typically be bound by an antibody or parts thereof (e.g., Fab monomers or dimers as described and provided herein, or F(ab')2 fragments or other Fab molecules as described herein and as known in the art).

The term "epitope" also refers to a site on an antigen to which the recognition molecule binds. Preferably, an epitope is a site on a molecule against which a recognition molecule, preferably an antibody will be produced and/or to which an antibody will bind. For example, an epitope can be recognized by a recognition molecule, particularly preferably by an antibody defining the epitope. A "linear epitope" is an epitope where an amino acid primary sequence comprises the epitope recognized. A linear epitope typically includes at least 3, and more usually, at least 5, for example, about 8 to about 10 amino acids in a unique sequence.

A "conformational epitope", in contrast to a linear epitope, is an epitope wherein the primary sequence of the amino acids comprising the epitope is not the sole defining component of the epitope recognized (e.g., an epitope wherein the primary sequence of amino acids is not necessarily recognized by the antibody defining the epitope). Typically a conformational epitope comprises an increased number of amino acids relative to a linear epitope. With regard to recognition of conformational epitopes, the recognition molecule recognizes a 3-dimensional structure of the antigen, preferably a peptide or protein or fragment thereof. For example, when a protein molecule folds to form a 3-dimensional structure, certain amino acids and/or the polypeptide backbone forming the conformational epitope become juxtaposed enabling the antibody to recognize the epitope. Methods of determining conformation of epitopes include but are not limited to, for example, x-ray crystallography 2-dimensional nuclear magnetic resonance spectroscopy and site-directed spin labelling and electron paramagnetic resonance spectroscopy.

An "antibody" when used herein is a protein comprising one or more polypeptides (comprising one or more binding domains, preferably antigen binding domains) substantially or partially encoded by immunoglobulin genes or fragments of immunoglobulin genes. The term "immunoglobulin" (Ig) is used interchangeably with "antibody" herein. The recognized immunoglobulin genes include the kappa, lambda, alpha, gamma, delta, epsilon and mu constant region genes, as well as myriad immunoglobulin variable region genes.

In particular, an "antibody" when used herein, is typically tetrameric glycosylated proteins composed of two light (L) chains of approximately 25 kDa each and two heavy (H) chains of approximately 50 kDa each. Two types of light chain, termed lambda and kappa, may be found in antibodies. Depending on the amino acid sequence of the constant domain of heavy chains, immunoglobulins can be assigned to five major classes: A, D, E, G, and M, and several of these may be further divided into subclasses (isotypes), e.g., IgG1, IgG2, IgG3, IgG4, IgA1, and IgA2 (for human beings). An IgM antibody consists of 5 of the basic heterotetramer unit along with an additional polypeptide called a J chain, and contains 10 antigen binding sites, while IgA antibodies comprise from 2-5 of the basic 4-chain units which can polymerize to form polyvalent assemblages in combination with the J chain. In the case of IgGs, the 4-chain unit is generally about 150,000 daltons.

Each light chain includes an N-terminal variable (V) domain (VL) and a constant (C) domain (CL). Each heavy chain includes an N-terminal V domain (VH), three or four C domains (CHs), and a hinge region. The constant domains are not involved directly in binding an antibody to an antigen.

The pairing of a VH and VL together forms a single antigen-binding site. The CH domain most proximal to VH is designated as CH1. Each L chain is linked to an H chain by one covalent disulfide bond, while the two H chains are linked to each other by one or more disulfide bonds depending on the H chain isotype. The VH and VL domains consist of four regions of relatively conserved sequences called framework regions (FR1, FR2, FR3, and FR4), which form a scaffold for three regions of hypervariable sequences (complementarity determining regions, CDRs). The CDRs contain most of the residues responsible for specific interactions of the antibody with the antigen. CDRs are referred to as CDR 1, CDR2, and CDR3. Accordingly, CDR constituents on the heavy chain are referred to as H1, H2, and H3, while CDR constituents on the light chain are referred to as L1, L2, and L3. The term "variable" refers to the portions of the immunoglobulin domains that exhibit variability in their sequence and that are involved in determining the specificity and binding affinity of a particular antibody (*i.e.* the "variable domain(s)"). Variability is not evenly distributed throughout the variable domains of antibodies; it is concentrated in sub-domains of each of the heavy and light chain variable regions. These sub-domains are called "hypervariable" regions or "complementarity determining regions" (CDRs). The more conserved (*i.e.* non-hypervariable) portions of the variable domains are called the "framework" regions (FRM). The variable domains of naturally occurring heavy and light chains each comprise four FRM regions, largely adopting a β- sheet configuration, connected by three hypervariable regions, which form loops connecting, and in some cases forming part of, the β -sheet structure. The hypervariable regions in each chain are held together in close proximity by the FRM and, with the hypervariable regions from the other chain, contribute to the formation of the antigen- binding site (after Kabat *et al.*). The constant domains are not directly involved in antigen binding, but exhibit various effector functions, such as, for example, antibody-dependent, cell-mediated cytotoxicity and complement activation.

The terms "CDR", and its plural "CDRs", refer to a complementarity determining region (CDR) of which three make up the binding character of a light chain variable region (CDRL1, CDRL2 and CDRL3; also referred to herein as VL-CDR, VL-CDR2, and VL-CDR3) and three make up the binding character of a heavy chain variable region (CDRH1, CDRH2 and CDRH3; also referred to herein as VH-CDR, VH-CDR2, and VH-CDR3). CDRs contribute to the functional activity of an antibody molecule and are separated by amino acid sequences that comprise scaffolding or framework regions. The exact definitional CDR boundaries and lengths are subject to different classification and numbering systems. CDRs may therefore be referred to by Kabat, Chothia, contact or any other boundary definitions, including the numbering system described herein. Despite differing boundaries, each of these systems has some degree of overlap in what constitutes the so called "hypervariable regions" within the variable sequences. CDR definitions according to these systems may therefore differ in length and boundary areas with respect to the adjacent framework region. See for example Kabat, Chothia, and/or MacCallum (Kabat *et al., loc. cit.*; Chothia et al., J Mol Biol (1987), 196: 901; and MacCallum et al., J Mol Biol (1996), 262: 732). However, the numbering in accordance with the so-called Kabat system is preferred.

In specific embodiments of the present invention, the first and the second Fab monomers may comprise specific VL and VH regions, each comprising one or more specific CDR regions. For example, in one specific embodiment of the present invention, said first Fab monomer comprises (A) the VH-CDR1 shown in SEQ ID NO: 1, the VH-CDR2 shown in SEQ ID NO: 2, the VH-CDR3 shown in SEQ ID NO: 3, the VL-CDR1 shown in SEQ ID NO: 4, the VL-CDR2 shown in SEQ ID NO: 5 and/or (preferably and) the VL-CDR3 shown in SEQ ID NO: 6, or (B) any one of or all sequences of (A), wherein at least 1, and up to 5, preferably up to 4, more preferably up to 3, more preferably up to 2, more preferably exactly 1 amino acid(s) are/is substituted, deleted or added (preferably substituted) compared to the respective amino acid sequences SEQ ID NOs. 1 to 6. In a specific embodiment of the present invention, said first Fab monomer comprises the VH-CDR1 shown in SEQ ID NO: 1, the VH-CDR2 shown in SEQ ID NO: 2, the VH-CDR3 shown in SEQ ID NO: 3, the VL-CDR1 shown in SEQ ID NO: 4, the VL-CDR2 shown in SEQ ID NO: 5 and the VL-CDR3 shown in SEQ ID NO: 6. In another specific embodiment of the present invention, said first Fab monomer comprises the VH-CDR1 shown in SEQ ID NO: 1, the VH-CDR2 shown in SEQ ID NO: 2, the VH-CDR3 shown in SEQ ID NO: 3, the VL-CDR1 shown in SEQ ID NO: 4, the VL-CDR2 shown in SEQ ID NO: 5 and/or (preferably and) the VL-CDR3 shown in SEQ ID NO: 6, wherein in any one of or all sequences according to SEQ ID NOs. 1 to 6, respectively, at least 1, and up to 5, preferably up to 4, more preferably up to 3, more preferably up to 2, more preferably exactly 1 amino acid(s) are/is substituted, and where at least one of or (preferably) all of said substitutions are/is (a) conservative or highly conservative substitution(s).

As used herein, "conservative" substitutions mean substitutions as listed as "Exemplary Substitutions" in Table I herein. "Highly conservative" substitutions as used herein mean substitutions as shown under the heading "Preferred Substitutions" in Table I herein.

**TABLE I Amino Acid Substitutions**

| Original | Exemplary Substitutions | Preferred Substitutions |
|---|---|---|
| Ala (A) | val; leu; ile | Val |
| Arg (R) | lys; gln; asn | lys |
| Asn (N) | gln; his; asp, lys; arg | gln |
| Asp (D) | glu; asn | glu |
| Cys (C) | ser; ala | ser |
| Gln (Q) | asn; glu | asn |
| Glu (E) | asp; gln | asp |
| Gly (G) | ala | ala |
| His (H) | asn; gln; lys; arg | arg |
| Ile (I) | leu; val; met; ala; phe; | leu |
| Leu (L) | norleucine; ile; val; met; ala; | ile |
| Lys (K) | arg; gin; asn | arg |
| Met (M) | leu; phe; ile | leu |
| Phe (F) | leu; val; ile; ala; tyr | tyr |
| Pro (P) | ala | ala |
| Ser (S) | thr | thr |
| Thr (T) | ser | ser |
| Trp (W) | tyr; phe | tyr |
| Tyr (Y) | trp; phe; thr; ser | Phe |
| Val (V) | ile; leu; met; phe; ala; | leu |

Unless specified herein otherwise, the term "position" when used in accordance with the present invention means the position of an amino acid within an amino acid sequence depicted herein. Unless specified herein otherwise, the term "corresponding" in this context also includes that a position may not only be determined by the number of the preceding nucleotides/amino acids.

The term "amino acid" or "amino acid residue" as used herein typically refers to an amino acid having its art recognized definition such as an amino acid selected from the group consisting of: alanine (Ala or A); arginine (Arg or R); asparagine (Asn or N); aspartic acid (Asp or D); cysteine (Cys or C); glutamine (Gln or Q); glutamic acid (Glu or E); glycine (Gly or G); histidine (His or H); isoleucine (He or I): leucine (Leu or L); lysine (Lys or K); methionine (Met or M); phenylalanine (Phe or F); pro line (Pro or P); serine (Ser or S); threonine (Thr or T); tryptophan (Trp or W); tyrosine (Tyr or Y); and valine (Val or V), although modified, synthetic, or rare amino acids may be used as desired. Generally, amino acids can be grouped as having a nonpolar side chain (e.g., Ala, Cys, He, Leu, Met, Phe, Pro, Val); a negatively charged side chain (e.g., Asp, Glu); a positively charged sidechain (e.g., Arg, His, Lys); or an uncharged polar side chain (e.g., Asn, Cys, Gln, Gly, His, Met, Phe, Ser, Thr, Trp, and Tyr).

In accordance with the present invention, the VL and/or the VH regions of the respective first and second Fab monomers may also comprise at their N-termini a secretion sequence. As generally known in the art, such secretion sequences may be relevant for allowing the synthesized Fab monomers to become secreted out of the Fab producing cell. For example, in context with the present invention, such secretion sequence at the N-terminus of the VL and/or the VH region of the first and/or second Fab monomer may comprise or consist of an amino acid sequence as shown in SEQ ID NO: 15 or 16, or comprise or consist of an amino acid sequence as shown in SEQ ID NO: 15 or 16, wherein at least 1, and up to 5, preferably up to 4, more preferably up to 3, more preferably up to 2, more preferably exactly 1 amino acid(s) are/is substituted, deleted or added (preferably substituted) compared to the respective amino acid sequences SEQ ID NOs. 15 or 16. In a specific embodiment of the present invention, such secretion sequence at the N-terminus of the VL and/or the VH region of the first and/or second Fab monomer may comprise or consist of an amino acid sequence as shown in SEQ ID NO: 15 or 16. In another specific embodiment of the present invention, such secretion sequence at the N-terminus of the VL and/or the VH region of the first and/or second Fab monomer may comprise or consist of an amino acid sequence as shown in SEQ ID NO: 15 or 16, wherein at least 1, and up to 5, preferably up to 4, more preferably up to 3, more preferably up to 2, more preferably exactly 1 amino acid(s) compared to the amino acid sequences as shown in SEQ ID NO: 15 or 16 are/is substituted, and where at least one of or (preferably) all of said substitutions are/is (a) conservative or highly conservative substitution(s) as defined herein.

In this context, in a specific embodiment of the present invention, the VL region of said first Fab monomer and the VL region of said second Fab monomer comprises at its N-terminus the secretion sequence shown in SEQ ID NO: 16. In another specific embodiment of the present invention, the VH region of said first Fab monomer and the VH region of said second Fab monomer comprises at its N-terminus the secretion sequence shown in SEQ ID NO: 16. In another specific embodiment of the present invention, the VL region of said first Fab monomer and the VH region of said second Fab monomer comprises at its N-terminus the secretion sequence shown in SEQ ID NO: 16. In another specific embodiment of the present invention, the VH region of said first Fab monomer and the VL region of said second Fab monomer comprises at its N-terminus the secretion sequence shown in SEQ ID NO: 16.

In this context, in another specific embodiment of the present invention, the VH region of said first Fab monomer and the VH region of said second Fab monomer comprises at its N-terminus the secretion sequence shown in SEQ ID NO: 15. In another specific embodiment of the present invention, the VL region of said first Fab monomer and the VL region of said second Fab monomer comprises at its N-terminus the secretion sequence shown in SEQ ID NO: 15. In another specific embodiment of the present invention, the VH region of said first Fab monomer and the VL region of said second Fab monomer comprises at its N-terminus the secretion sequence shown in SEQ ID NO: 15. In another specific embodiment of the present invention, the VL region of said first Fab monomer and the VH region of said second Fab monomer comprises at its N-terminus the secretion sequence shown in SEQ ID NO: 15.

In a more specific embodiment of the present invention, the VL region of said first Fab monomer and the VL region of said second Fab monomer comprises at its N-terminus the secretion sequence shown in SEQ ID NO: 16, and the VH region of said first Fab monomer and the VH region of said second Fab monomer comprises at its N-terminus the secretion sequence shown in SEQ ID NO: 15. In another more specific embodiment of the present invention, the VH region of said first Fab monomer and the VH region of said second Fab monomer comprises at its N-terminus the secretion sequence shown in SEQ ID NO: 16, and the VL region of said first Fab monomer and the VL region of said second Fab monomer comprises at its N-terminus the secretion sequence shown in SEQ ID NO: 15. In another more specific embodiment of the present invention, the VL region of said first Fab monomer and the VH region of said second Fab monomer comprises at its N-terminus the secretion sequence shown in SEQ ID NO: 16, and the VH region of said first Fab monomer and the VL region of said second Fab monomer comprises at its N-terminus the secretion sequence shown in SEQ ID NO: 15. In another specific embodiment of the present invention, the VH region of said first Fab monomer and the VL region of said second Fab monomer comprises at its N-terminus the secretion sequence shown in SEQ ID NO: 16, and the VL region of said first Fab monomer and the VH region of said second Fab monomer comprises at its N-terminus the secretion sequence shown in SEQ ID NO: 15.

In a very specific embodiment of the present invention, said first and/or second Fab monomer comprises the VH region shown in SEQ ID NO: 7 and/or (preferably and) the VL region shown in SEQ ID NO: 8. In another specific embodiment of the present invention, said first and/or second Fab monomer comprises the VH region shown in SEQ ID NO: 7 and/or (preferably and) the VL region shown in SEQ ID NO: 8 wherein at least 1, and up to 5, preferably up to 4, more preferably up to 3, more preferably up to 2, more preferably exactly 1 amino acid(s) are/is substituted, deleted or added (preferably substituted) compared to the respective amino acid sequences SEQ ID NOs. 7 or 8. In the latter context, in one embodiment of the present invention, said first and/or second Fab monomer comprises the VH region shown in SEQ ID NO: 7 and/or (preferably and) the VL region shown in SEQ ID NO: 8 wherein at least 1, and up to 5, preferably up to 4, more preferably up to 3, more preferably up to 2, more preferably exactly 1 amino acid(s) are/is substituted compared to the respective amino acid sequences SEQ ID NOs. 7 or 8, and where at least one of or (preferably) all of said substitutions are/is (a) conservative or highly conservative substitution(s) as defined herein.

In another very specific embodiment of the present invention, said first and/or second Fab monomer comprises the VH region encoded by the nucleotide sequence shown in SEQ ID NO: 9 and/or (preferably and) the VL region encoded by the nucleotide sequence shown in SEQ ID NO: 10, or said first and/or second Fab monomer comprises the VH region encoded by the nucleotide sequence shown in SEQ ID NO: 9 and/or (preferably and) the VL region encoded by the nucleotide sequence shown in SEQ ID NO: 10, wherein 1 to 30, preferably 1 to 21, more preferably 1 to 12, more preferably 1 to 6 nucleotides are substituted, added or deleted (preferably substituted) compared to the respective nucleic acid sequences of SEQ ID NO. 9 or 10, respectively. In the latter case, where 1 to 30, preferably 1 to 21, more preferably 1 to 12, more preferably 1 to 6 nucleotides are substituted compared to the respective nucleic acid sequences of SEQ ID NO. 9 or 10, respectively, such substitutions are preferably conservative, highly conservative, or - more preferably - silent substitutions.

As used herein, "silent" substitutions or mutations mean base substitutions within a nucleic acid sequence which do not change the respective amino acid sequence encoded by the nucleic acid sequence. "Conservative" substitutions mean substitutions as listed as "Exemplary Substitutions" in Table I. "Highly conservative" substitutions as used herein mean substitutions as shown under the heading "Preferred Substitutions" in Table I.

As used herein, unless specifically defined otherwise, the term "nucleic acid" or "nucleic acid molecule" is used synonymously with "oligonucleotide", "nucleic acid strand", or the like, and means a polymer comprising one, two, or more nucleotides, e.g., single- or double stranded.

Likewise, as used herein, the terms "polynucleotide", "nucleic acid" and "nucleic acid molecule" are to be construed synonymously. Generally, nucleic acid molecules may comprise *inter alia* DNA molecules, RNA molecules, oligonucleotide thiophosphates, substituted ribo-oligonucleotides or PNA molecules. Furthermore, the term "nucleic acid molecule" may refer to DNA or RNA or hybrids thereof or any modification thereof that is known in the art (see, e.g., US 5525711, US 471 1955, US 5792608 or EP 302175 for examples of modifications). The polynucleotide sequence may be single- or doublestranded, linear or circular, natural or synthetic, and without any size limitation. For instance, the polynucleotide sequence may be genomic DNA, cDNA, mitochondrial DNA, mRNA, antisense RNA, ribozymal RNA or a DNA encoding such RNAs or chimeroplasts (Gamper, Nucleic Acids Research, 2000, 28, 4332 - 4339). Said polynucleotide sequence may be in the form of a vector, plasmid or of viral DNA or RNA. Also described herein are nucleic acid molecules which are complementary to the nucleic acid molecules described above and nucleic acid molecules which are able to hybridize to nucleic acid molecules described herein. A nucleic acid molecule described herein may also be a fragment of the nucleic acid molecules in context of the present invention. Particularly, such a fragment is a functional fragment. Examples for such functional fragments are nucleic acid molecules which can serve as primers.

In a further specific embodiment of the present invention, said first and/or second Fab monomer comprises the VH region shown in SEQ ID NO: 11 and/or (preferably and) the VL region shown in SEQ ID NO: 12. In another specific embodiment of the present invention, said first and/or second Fab monomer comprises the VH region shown in SEQ ID NO: 11 and/or (preferably and) the VL region shown in SEQ ID NO: 12 wherein at least 1, and up to 5, preferably up to 4, more preferably up to 3, more preferably up to 2, more preferably exactly 1 amino acid(s) are/is substituted, deleted or added (preferably substituted) compared to the respective amino acid sequences SEQ ID NOs. 11 or 12. In the latter context, in one embodiment of the present invention, said first and/or second Fab monomer comprises the VH region shown in SEQ ID NO: 11 and/or (preferably and) the VL region shown in SEQ ID NO: 12 wherein at least 1, and up to 5, preferably up to 4, more preferably up to 3, more preferably up to 2, more preferably exactly 1 amino acid(s) are/is substituted compared to the respective amino acid sequences SEQ ID NOs. 11 or 12, and where at least one of or (preferably) all of said substitutions are/is (a) conservative or highly conservative substitution(s) as defined herein.

In a further specific embodiment of the present invention, said first and/or second Fab monomer comprises the VH region encoded by the nucleotide sequence shown in SEQ ID NO: 13 and the VL region encoded by the nucleotide sequence shown in SEQ ID NO: 14. In a further specific embodiment of the present invention, said first and/or second Fab monomer comprises the VH region encoded by the nucleotide sequence shown in SEQ ID NO: 13 and the VL region encoded by the nucleotide sequence shown in SEQ ID NO: 14, wherein said first and/or second Fab monomer comprises the VH region encoded by the nucleotide sequence shown in SEQ ID NO: 13 and/or (preferably and) the VL region encoded by the nucleotide sequence shown in SEQ ID NO: 14, wherein 1 to 30, preferably 1 to 21, more preferably 1 to 12, more preferably 1 to 6 nucleotides are substituted, added or deleted (preferably substituted) compared to the respective nucleic acid sequences of SEQ ID NO. 13 or 14, respectively. In the latter case, where 1 to 30, preferably 1 to 21, more preferably 1 to 12, more preferably 1 to 6 nucleotides are substituted compared to the respective nucleic acid sequences of SEQ ID NO. 13 or 14, respectively, such substitutions are preferably conservative, highly conservative, or - more preferably - silent substitutions.

The present invention further relates to a dimer as described and provided herein which is obtainable by
(a) culturing a suitable cell (e.g. CHO or HEK293 cells) expressing a first and a second Fab monomer as defined herein until stationary phase is reached;
(b) optionally adding an oxidant (e.g. O₂, 1 mM H₂O₂, and/or 1 mM MnO₄⁻) to the cell culture; and
(c) harvesting said dimer.

As used herein, "stationary phase" may mean that there is no further substantial (e.g. not more than about 2%, preferably not more than about 1% over 2 hours) net increase in cell numbers (e.g., due to a balance between dividing and dying cells), preferably no further net increase in cell numbers.

Furthermore, in accordance with the present invention, the first and/or second Fab monomer may be coupled to other compounds, e.g., for diagnosis or therapeutic purposes. Such compounds may comprise, e.g., labelling groups, toxins, or anti-tumor drugs (e.g., fluorochromes, enzymes (like peroxidase), radionuclides, rizin, ozogamicin, emtansine, monimethylauristin E, α-amanitin). Such coupling may be conducted chemically after expression of the antibody or antigen to the site of attachment, or the coupling product may be engineered into the antibody or antigen of the invention at the DNA level. The DNAs are then expressed in a suitable host system as described herein below, and the expressed proteins are collected and renatured, if necessary. Coupling may be achieved via a linker known in the state of the art. In particular, different linkers that release the toxin, or an anti-tumor drug under acidic or reducing conditions or upon exposure to specific proteases may be employed with this technology.

In certain aspects, it may be desirable, that the labelling group, toxin, or an anti-tumor drug is attached by spacer arms of various lengths to reduce potential steric hindrance.

The present invention further relates to compounds comprising a dimer as described and provided herein. In accordance with the present invention, such compounds comprising a dimer as described and provided herein may additionally also comprise one or more Fab monomers (non-dimerized) as described and provided herein. In one embodiment of the present invention, such compound may be for use as a medicament. For example, said compound comprising a dimer as described and provided herein may be a pharmaceutical composition.

Unless specified otherwise, when referring to a "dimer" herein, a Fab dimer as described herein is meant. Likewise, when referring to a "monomer", a Fab monomer as described herein is meant.

Generally, in context with the present invention, the compound described and provided herein may comprise one or more dimers as described herein, and one or more monomers as described herein. For example, in a composition described and provided herein, the ratio between dimers and monomers can range from about 100:0, about 99:1, about 90:10, about 80:20, about 70:30, about 60:40, about 50:50, about 40:60, about 30:70, about 20:80, about 10:90, or about 1:99. As a specific example, the ratio dimer to monomer may be about 40:60.

The (pharmaceutical) composition is preferably administered to mammals such as domestic and pet animals. Most preferred it is administered to humans. The pharmaceutical compositions described herein can be administered to the subject at a suitable dose. The pharmaceutical composition for use in accordance with the present invention can be formulated in conventional manner according to methods found in the art, using one or more physiological carriers or excipient, see, for example Ansel et al., "Pharmaceutical Dosage Forms and Drug Delivery Systems", 7th edition, Lippincott Williams & Wilkins Publishers, 1999. The (pharmaceutical) composition may, accordingly, be administered orally, parenterally, such as subcutaneously, intravenously, intramuscularly, intraperitoneally, intrathecally, transdermally, transmucosally, subdurally, locally or topically via iontopheresis, sublingually, by inhalation spray, aerosol or rectally and the like in dosage unit formulations optionally comprising conventional pharmaceutically acceptable excipients. For oral administration, the (pharmaceutical) composition of the invention can take the form of, for example, tablets or capsules prepared by conventional means with pharmaceutical acceptable excipients such as binding agents (e.g., pregelatinised maize starch, polyvinylpyrrolidone, hydroxypropyl methylcellulose), fillers (e.g., lactose, microcrystalline cellulose, calcium hydrogen phosphate), lubricants (e.g., magnesium stearate, talc, silica), disintegrants (e.g., potato starch, sodium starch glycolate), or wetting agents (e.g., sodium lauryl sulphate). The (pharmaceutical) composition can be administered with a physiologically acceptable carrier to a patient, as described herein. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the (pharmaceutical) composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium ion, dried skim milk, glycerol, propylene, glycol, water, ethanol and the like. The composition, if desired, can also contain minor amounts emulsifying agents, or pH buffering agents. These compositions can be in the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin. Such compositions will contain a therapeutically effective amount of the aforementioned compounds, preferably in purified form, together with a suitable amount of carrier so as to provide the form for proper administration to the patient. The formulation should suit the mode of administration.

The pharmaceutical composition of the invention can be administered as sole active agent or can be administered in combination with other agents, preferably ones that are known in the art to be suitable for treatment of the disease in question.

In accordance with the present invention, the dimer as described and provided herein, the compound comprising such dimers (and one or more Fab monomers (non-dimerized)) as described and provided herein, may also be for use in a method for treating or inhibiting hypoxia, a solid tumor, or an eye disease.

In context with the present invention, for example, hypoxia may be selected from tumor hypoxia, neuronal hypoxia, cerebral hypoxia, stenosis and ischemia. In this regard the term "hypoxia" refers to a pathological condition in which the body as a whole (generalized hypoxia) or a region of the body (tissue hypoxia) is deprived of adequate oxygen supply. For example, a mismatch between oxygen supply and its demand at the cellular level may result in a hypoxic condition. Hypoxia in which there is complete deprivation of oxygen supply is referred to as anoxia and therefore is embraced by the umbrella term hypoxia.

The term "solid tumor" in accordance with the invention defines an abnormal mass of tissue that usually does not contain cysts or liquid areas. Solid tumors may be benign (not cancer), or malignant (often referred to in the art as cancer). Different types of solid tumors are named for the type of cells that form them. In context with the present invention, for example, a solid tumor may be selected from sarcomas, glioma, carcinoma, mesotheliom, lymphoma, kidney tumor, lung tumor, mammary tumor, cervix tumor, ovarian tumor, colorectal tumor, liver tumor, prostate tumor, pancreas tumor and head and neck tumor. In one embodiment of the present invention, a solid tumor is a glioma or lung tumor. In a specific embodiment of the present invention, a solid tumor is a glioma.

The term "eye disease" as used herein refers to a pathological condition or injury of the eye including its adnexa. An eye disease may for example involve the clouding or pacification of the natural lens of the eye, swelling of the macula for example resulting from leakage and accumulation of fluid or of the central retina, small accumulations of hyaline bodies underneath the retina, damages of the optic nerve, degeneration of the cells of the macula lutea, loss of vision or inflammation of the conjunctiva and cornea of the eye, and the formation of scar tissue. In context with the present invention, for example, an eye disease may be selected from ocular hypertension, glaucoma, macular degeneration, age-related macular degeneration, uveitis, retinitis, X-linked retinoschisis and hypertensive retinopathy.

The embodiments which characterize the present invention are described herein, shown in the Figures, illustrated in the Examples, and reflected in the claims.

The present invention is also characterized by the following items:
(1) A dimer composed of a first Fab monomer and a second Fab monomer, each Fab monomer comprising a VH and a VL region, wherein
   (i) the VL region of the first Fab monomer and the VL region of the second Fab monomer are covalently linked by a disulfide bond between an additional non-naturally occurring cysteine residue at the N-terminus of the VL region of the first Fab monomer and the N-terminus of the VL region of the second Fab monomer;
   (ii) the VH region of the first Fab monomer and the VH region of the second Fab monomer are covalently linked by a disulfide bond between an additional non-naturally occurring cysteine residue at the N-terminus of the VH region of the first Fab monomer and the N-terminus of the VH region of the second Fab monomer;
   (iii) the VL region of the first Fab monomer and the VH region of the second Fab monomer are covalently linked by a disulfide bond between an additional non-naturally occurring cysteine residue at the N-terminus of the VL region of the first Fab monomer and the N-terminus of the VH region of the second Fab monomer; or
   (iv) the VH region of the first Fab monomer and the VL region of the second Fab monomer are covalently linked by a disulfide bond between an additional non-naturally occurring cysteine residue at the N-terminus of the VH region of the first Fab monomer and the N-terminus of the VL region of the second Fab monomer.
(2) The dimer of item 1, wherein the non-naturally occurring cysteine residue at the N-terminus of the VL region or VH region is at amino acid position 1 or amino acid position 2, wherein amino acids are counted from the N-terminus of the VL region or VH region, respectively, with position 1 being the first amino acid at the N-terminus.
(3) The dimer of item 1 or 2, wherein said first Fab monomer and said second Fab monomer are the same or are different.
(4) The dimer of any one of the preceding items, wherein said first Fab monomer and said second Fab monomer are directed against the same target.
(5) The dimer of any one of items 1 to 3, wherein said first Fab monomer is directed against a first target and said second Fab monomer is directed against a second target, said first and second target being different.
(6) The dimer of any one of the preceding items, wherein said first Fab monomer comprises the VH-CDR1 shown in SEQ ID NO: 1, the VH-CDR2 shown in SEQ ID NO: 2, the VH-CDR3 shown in SEQ ID NO: 3, the VL-CDR1 shown in SEQ ID NO: 4, the VL-CDR2 shown in SEQ ID NO: 5 and the VL-CDR3 shown in SEQ ID NO: 6.
(7) The dimer of any one of the preceding items, wherein the VL region of said first Fab monomer and the VL region of said second Fab monomer comprises at its N-terminus the secretion sequence shown in SEQ ID NO: 16.
(8) The dimer of any one of the preceding items, wherein the VH region of said first Fab monomer and the VH region of said second Fab monomer comprises at its N-terminus the secretion sequence shown in SEQ ID NO: 16.
(9) The dimer of any one of the preceding items, wherein the VL region of said first Fab monomer and the VH region of said second Fab monomer comprises at its N-terminus the secretion sequence shown in SEQ ID NO: 16.
(10) The dimer of any one of the preceding items, wherein the VH region of said first Fab monomer and the VL region of said second Fab monomer comprises at its N-terminus the secretion sequence shown in SEQ ID NO: 16.
(11) The dimer of item 7, wherein the VH region of said first Fab monomer and the VH region of said second Fab monomer comprises at its N-terminus the secretion sequence shown in SEQ ID NO: 15.
(12) The dimer of item 8, wherein the VL region of said first Fab monomer and the VL region of said second Fab monomer comprises at its N-terminus the secretion sequence shown in SEQ ID NO: 15
(13) The dimer of item 9, wherein the VH region of said first Fab v and the VL region of said second Fab monomer comprises at its N-terminus the secretion sequence shown in SEQ ID NO: 15.
(14) The dimer of item 10, wherein the VL region of said first Fab monomer and the VH region of said second Fab monomer comprises at its N-terminus the secretion sequence shown in SEQ ID NO: 15.
(15) The dimer of any one of the preceding items, wherein said first Fab monomer comprises the VH region shown in SEQ ID NO: 7 and the VL region shown in SEQ ID NO: 8.
(16) The dimer of any one of the preceding items, wherein said first Fab monomer comprises the VH region encoded by the nucleotide sequence shown in SEQ ID NO: 9 and the VL region encoded by the nucleotide sequence shown in SEQ ID NO: 10.
(17) The dimer of any one of the preceding items, wherein said first Fab monomer comprises the VH region shown in SEQ ID NO: 11 and the VL region shown in SEQ ID NO: 12.
(18) The dimer of any one of the preceding items, wherein said first Fab monomer comprises the VH region encoded by the nucleotide sequence shown in SEQ ID NO: 13 and the VL region encoded by the nucleotide sequence shown in SEQ ID NO: 14.
(19) The dimer of any one of the preceding items, which is obtainable by
   (a) culturing a CHO cell expressing a first and a second Fab monomer as defined in any one of items 1 to 18 until stationary phase is reached;
   (b) optionally adding an oxidant to the CHO cell culture; and
   (c) harvesting said dimer.
(20) The dimer of any one of the preceding items, wherein the first and/or second Fab monomer is coupled to
   (a) a labelling group,
   (b) a toxin, or
   (c) an anti-tumor drug.
(21) A composition comprising the dimer of any one of items 1 to 20.
(22) The composition of clam 21, further comprising Fab monomers as defined in any one of items 1 to 20.
(23) The dimer of any one of items 1 to 20 or the composition of item 21 or 22 for use as a medicament.
(24) The dimer of any one of items 1 to 20 or the composition of item 21 or 22 for use in a method for treating or inhibiting hypoxia, a solid tumor, or an eye disease.
(25) The dimer or composition of item 24, wherein said hypoxia is selected from tumor hypoxia, neuronal hypoxia, cerebral hypoxia, stenosis and ischemia.
(26) The dimer or composition of item 24, wherein the solid tumor is selected from sarcomas, glioma, carcinoma, mesotheliom, lymphoma, kidney tumor, lung tumor, mammary tumor, cervix tumor, ovarian tumor, colorectal tumor, liver tumor, prostate tumor, pancreas tumor and head and neck tumor.
(27) The dimer or composition of item 24, wherein said eye disease is selected from ocular hypertension, glaucoma, macular degeneration, age-related macular degeneration, uveitis, retinitis, X-linked retinoschisis and hypertensive retinopathy.

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

In each instance herein any of the terms "comprising", "consisting essentially of" and "consisting of" may be replaced with either of the other two terms.

It should be understood that this invention is not limited to the particular methodology, protocols, and reagents, etc., described herein and as such can vary. The terminology used herein is for the purpose of describing particular embodiments only, and is not intended to limit the scope of the present invention, which is defined solely by the claims.

All publications and patents cited throughout the text of this specification (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention. To the extent the material incorporated by reference contradicts or is inconsistent with this specification, the specification will supersede any such material.

### Figures

The Figures show:

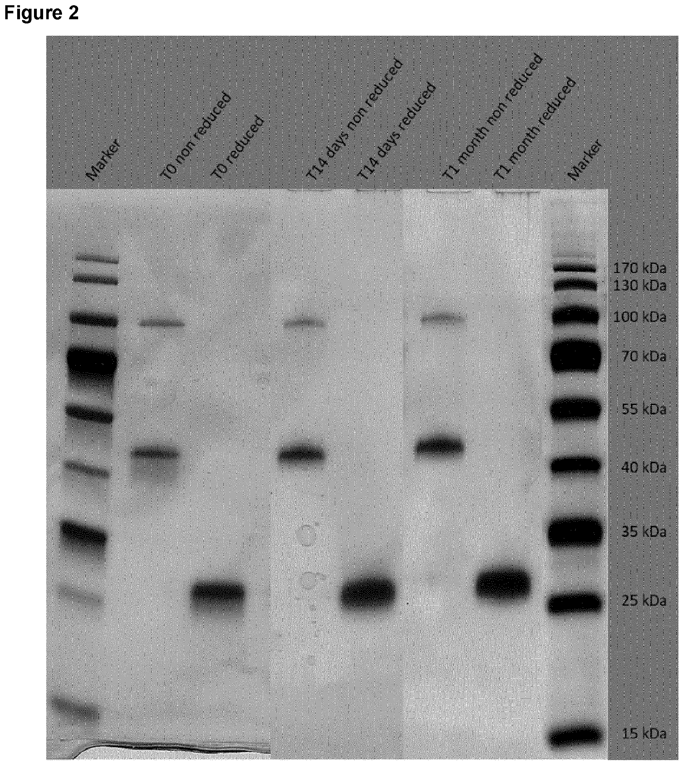

- **Figure 1:**: Vector-Structure. HitbasisHygro_ch6A10_LC and HitbasisNeo_ch6A10_HC vectors Translation of HitbasisHygro_ch6A10_LC: 
MSVPTQVLGLLLLWLTDARCPVDIVLTQSPATLSVTPGESVSLSCRASQGIS
TSIHWYQQKSNESPRLLIKFASQSISGIPSRFSGSGSGTDFTLSINRVESEDF
SVYFCQQTYSLPYTFGAGTKLELKRTVAAPSVFIFPPSDEQLKSGTASVVCLL
NNFYPREAKVQWKVDNALQSGNSQESVTEQDSKDSTYSLSSTLTLSKADYE
KHKVYACEVTHQGLSSPVTKSFNRGEC (SEQ ID NO: 12)
(bold = predicted signal (secretion) sequence )
 **Figure 2:** SDS-PAGE. Reducing SDS-PAGE analysis of 6A10 Fab dimers showed a characteristic band of about 25 - 30 kDa, corresponding to the light and heavy chain of 6A10 Fab fragment. Under non-reducing conditions bands of about 50 kDa and 100 kDa are detected, corresponding to the precursor monomer and a homo-dimer form.

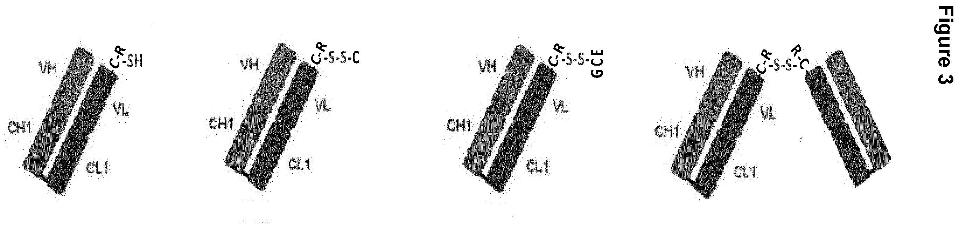

**Figure 3:** "Intact test" via LC-ESI-TOF. Further analysis of monomer and dimer forms of the precursor is done by LC-ESI-TOF. Homo- dimers are formed via a disulfide bridge of additional cysteine at the N-terminus of the light chain. In addition, different forms of the monomer with and without additional a cysteine or glutathione are detected in changing ratios.

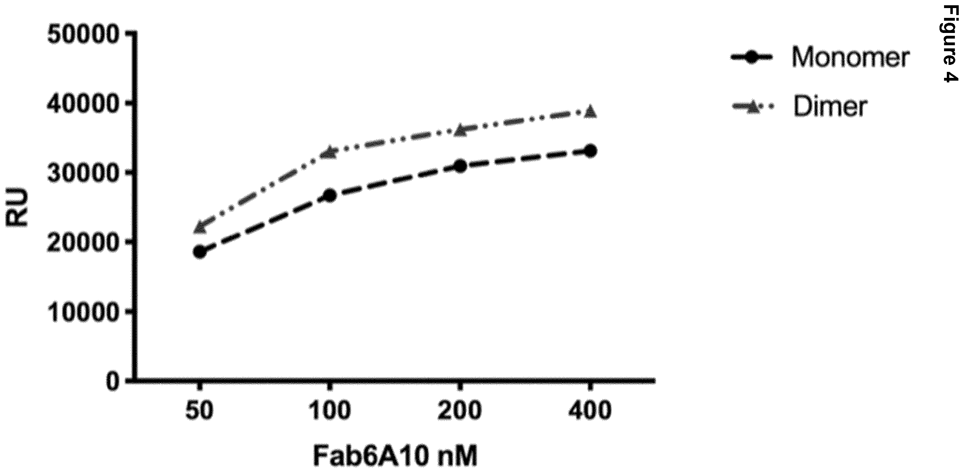

**Figure 4:** Binding of monomers and dimers to CA12-positive human A549 lung cancer cells. Flow cytometry revealed no significantly different binding behavior for Monomers and Dimers at pH 7.4. 50.000 A549 cells were incubated with a serial dilution of Monomers or Dimers (6A10). After washing, cells were incubated with a Cy5-labeled secondary antibody (donkey anti-human IgG light and heavy chain). Binding of the secondary antibody was analyzed with a BD FACSCanto and the DIVA software. RU = relative light units.

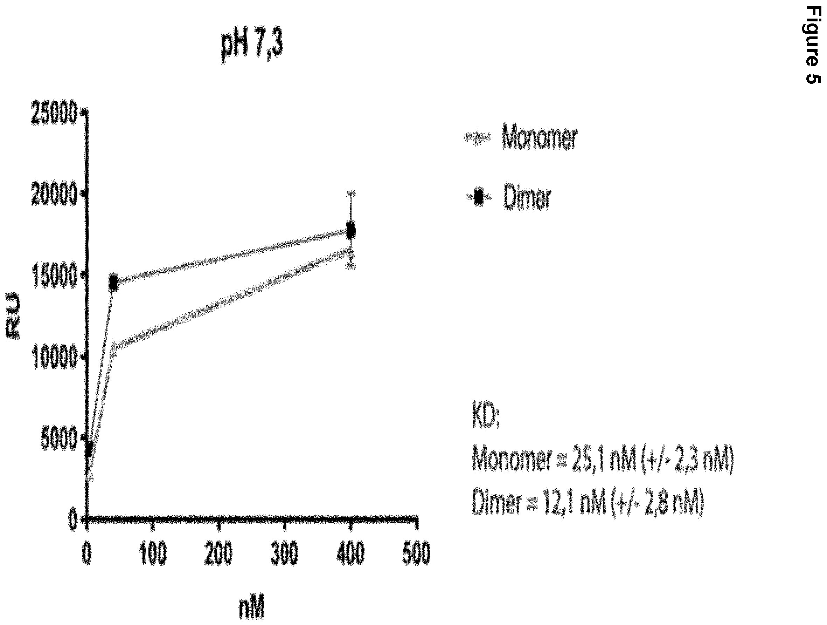

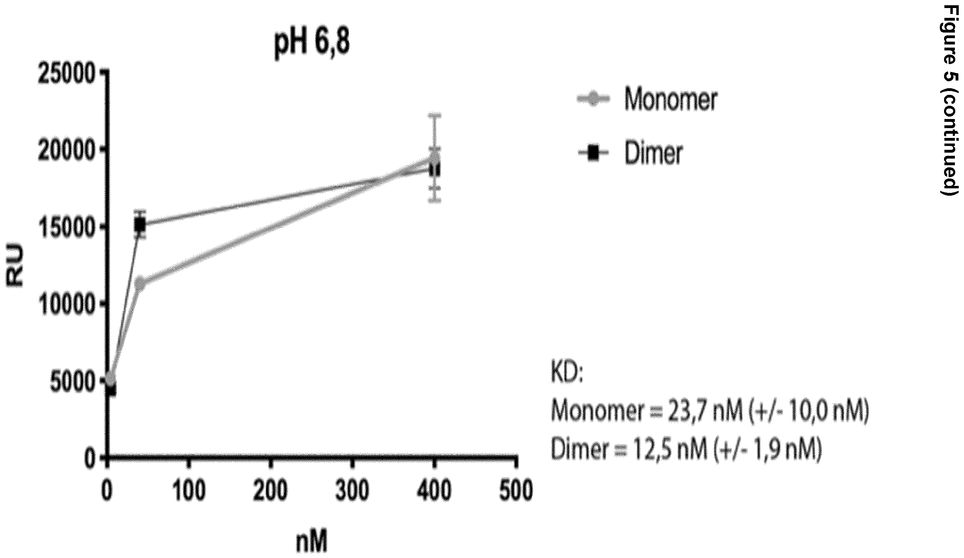

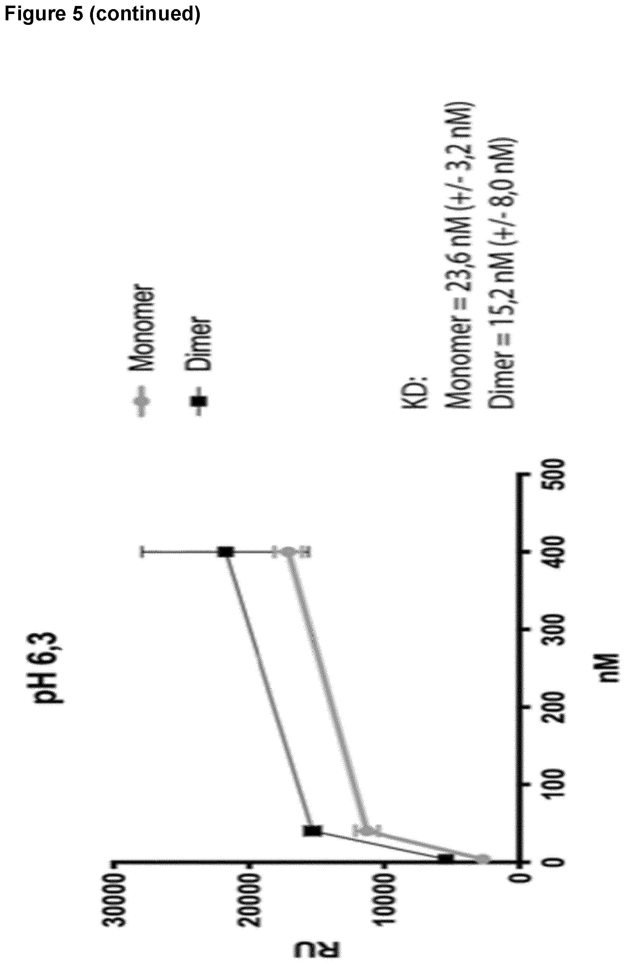

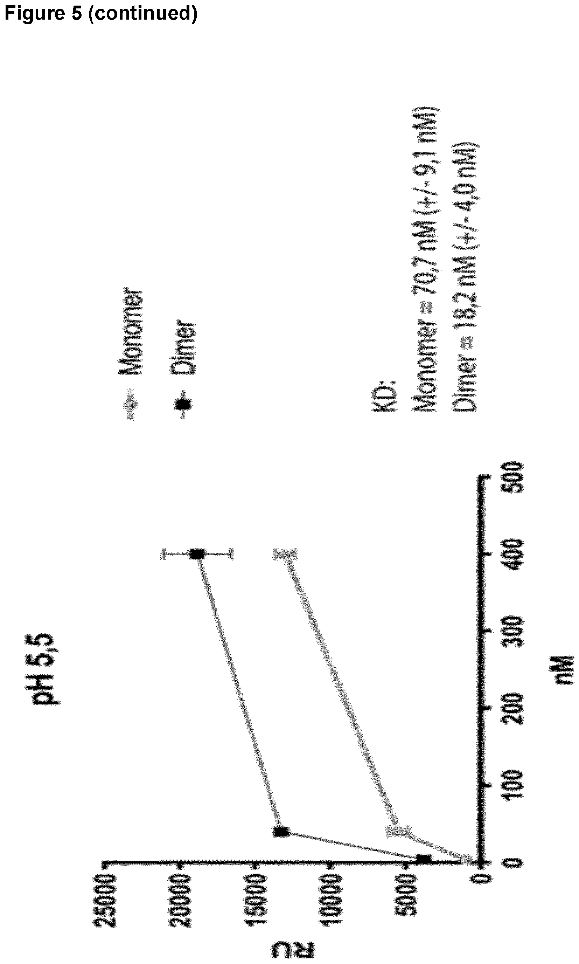

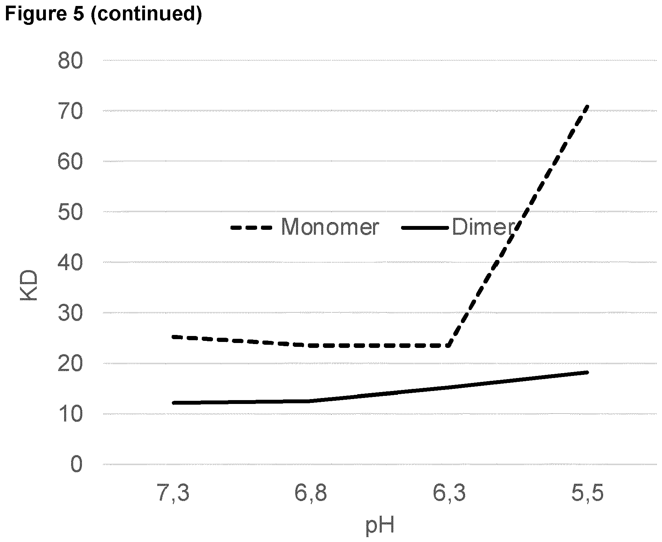

**Figure 5:** Flow cytometry. Flow cytometry with revealed a drastically reduced binding behavior for Monomers but not for Dimers (610A) in an acidic environment (pH 5.5) as it is typical for solid tumors.

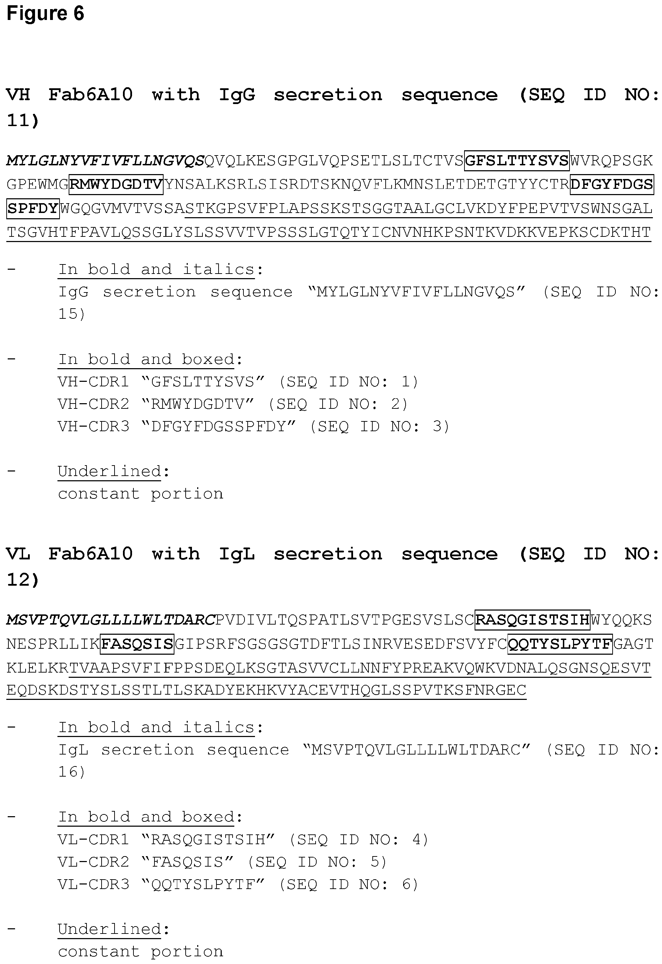

**Figure 6:** shows the amino acid sequence of the VH of Fab6A10 with IgG secretion sequence and the amino acid sequence of the VL of Fab6A10 with IgL secretion sequence

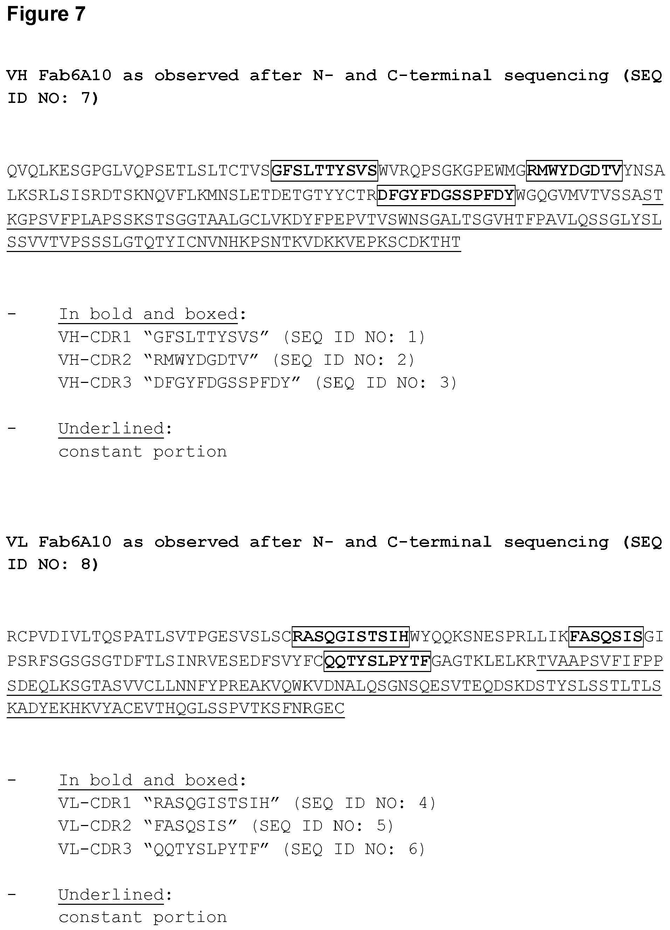

**Figure 7:** shows the amino acid sequence of the VH of Fab6A10 after N- and C-terminal sequencing and the amino acid sequence of the VL of Fab6A10 after N- and C-terminal sequencing

The present application also provides the following sequences referred to in the present application, for which also a sequence protocol was generated and which is part of the present application. However, in case of conflict between the following sequences and the sequences shown in the sequence listing, the following sequences supersede:
SEQ ID NO: 1 - VH-CDR1 of Fab6A10: GFSLTTYSVS
SEQ ID NO: 2 - VH-CDR2 of Fab6A10: RMWYDGDTV
SEQ ID NO: 3 - VH-CDR3 of Fab6A10: DFGYFDGSSPFDY
SEQ ID NO: 4 - VL-CDR1 of Fab6A10: RASQGISTSIH
SEQ ID NO: 5 - VL-CDR2 of Fab6A10: FASQSIS
SEQ ID NO: 6 - VL-CDR3 of Fab6A10: QQTYSLPYTF
SEQ ID NO: 7 - VH of Fab6A10: 
QVQLKESGPGLVQPSETLSLTCTVSGFSLTTYSVSWVRQPSGKGPEWMGRMWYDGDTV
YNSALKSRLSISRDTSKNQVFLKMNSLETDETGTYYCTRDFGYFDGSSPFDYWGQGVMVT
VSSASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKKVEPKSCDKTHT
(CDRs in bold and boxed, constant portion underlined)
SEQ ID NO: 8 - VL of Fab6A10: 
RCPVDIVLTQSPATLSVTPGESVSLSCRASQGISTSIHWYQQKSNESPRLLIKFASQSISGIP
SRFSGSGSGTDFTLSINRVESEDFSVYFCQQTYSLPYTFGAGTKLELKRTVAAPSVFIFPPS
DEQLKSGTASVVCLLNNFYPREAKVQWKVDNALQSGNSQESVTEQDSKDSTYSLSSTLTLS
KADYEKHKVYACEVTHQGLSSPVTKSFNRGEC
(CDRs in bold and boxed, constant portion underlined)
SEQ ID NO: 9 - Nucl. Acid of VH of Fab6A10: 
CAGGTCCAGCTGAAGGAGTCCGGTCCAGGTCTGGTCCAGCCTAGTGAGACCCTGTCAC
TGACTTGCACCGTGTCAGGATTCTCCCTGACCACATACAGTGTCTCATGGGTGAGACAG
CCTTCCGGGAAGGGTCCAGAATGGATGGGGAGGATGTGGTACGACGGCGACACAGTG
TATAACAGCGCCCTGAAATCTAGGCTGTCCATCAGCCGGGACACTTCTAAGAACCAGGT
GTTTCTGAAAATGAATTCCCTGGAGACTGATGAAACAGGCACTTACTATTGCACCAGGG
ACTTCGGGTACTTTGATGGTTCCAGCCCATTCGACTATTGGGGCCAGGGAGTGATGGTC
ACCGTGTCTAGTGCTAGTACAAAGGGGCCCAGCGTGTTTCCACTGGCACCCTCATCCAA
ATCTACTAGTGGAGGAACCGCCGCTCTGGGCTGTCTGGTGAAGGATTATTTCCCCGAG
CCTGTCACCGTGAGCTGGAATTCTGGCGCACTGACAAGCGGAGTCCACACTTTTCCAG
CCGTGCTGCAGAGCTCTGGCCTGTACTCCCTGAGTTCAGTGGTCACAGTCCCCTCCAG
CTCTCTGGGAACCCAGACATACATCTGTAACGTGAACCATAAGCCTAGCAACACTAAGG
TGGACAAAAAGGTGGAGCCAAAATCCTGCGACAAAACCCATACATAA
SEQ ID NO: 10 - Nucl. Acid of VL of Fab6A10: 
AGATGTCCCGTCGATATTGTGCTGACCCAGAGCCCAGCCACACTGAGTGTGACTCCCG
GAGAGTCTGTCAGTCTGTCATGCAGGGCTAGCCAGGGGATCTCCACCAGCATTCACTG
GTACCAGCAGAAGAGTAACGAATCACCCAGGCTGCTGATCAAATTCGCCTCTCAGAGTA
TCTCAGGAATTCCATCTCGGTTCTCCGGCAGCGGATCTGGGACTGACTTTACCCTGTCC
ATTAATAGAGTGGAGAGCGAAGATTTCTCTGTCTATTTTTGCCAGCAGACATACAGCCTG
CCCTATACCTTCGGTGCAGGCACAAAGCTGGAGCTGAAACGCACTGTGGCCGCTCCTA
GCGTCTTCATCTTTCCCCCTTCTGACGAACAGCTGAAGAGTGGTACAGCTTCAGTGGTC
TGTCTGCTGAACAATTTTTACCCACGAGAGGCAAAGGTGCAGTGGAAAGTCGATAACGC
CCTGCAGTCCGGCAATAGCCAGGAGTCTGTGACTGAACAGGACAGTAAGGATTCAACC
TATTCCCTGTCCAGCACCCTGACACTGTCCAAAGCTGACTACGAGAAGCATAAAGTGTA
TGCATGTGAAGTCACCCACCAGGGGCTGTCATCACCCGTCACAAAATCATTCAATAGGG
GGGAGTGCTAA
SEQ ID NO: 11 - VH of Fab6A10 with IgG secretion sequence: 
MYLGLNYVFIVFLLNGVQSQVQLKESGPGLVQPSETLSLTCTVSGFSLTTYSVSWVRQPSG
KGPEWMGRMWYDGDTVYNSALKSRLSISRDTSKNQVFLKMNSLETDETGTYYCTRDFGY
FDGSSPFDYWGQGVMVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSNTKVDKKVEPK
SCDKTHT
(secretion sequence in italics and bold, CDRs in bold and boxed, constant portion
underlined)
SEQ ID NO: 12 - VL of Fab6A10 with IgL secretion sequence: 
MSVPTQVLGLLLLWLTDARCPVDIVLTQSPATLSVTPGESVSLSCRASQGISTSIHWYQQK
SNESPRLLIKFASQSISGIPSRFSGSGSGTDFTLSINRVESEDFSVYFCQQTYSLPYTFGAGT
KLELKRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
(secretion sequence in italics and bold and boxed, CDRs in bold and boxed, constant portion
underlined)
SEQ ID NO: 13 - Nucl. Acid of VH Fab6A10 with IgG secretion sequence: 
ATGTATCTGGGTCTGAACTATGTCTTTATCGTCTTTCTGCTGAACGGGGTCCAGTCACAG
GTCCAGCTGAAGGAGTCCGGTCCAGGTCTGGTCCAGCCTAGTGAGACCCTGTCACTGA
CTTGCACCGTGTCAGGATTCTCCCTGACCACATACAGTGTCTCATGGGTGAGACAGCCT
TCCGGGAAGGGTCCAGAATGGATGGGGAGGATGTGGTACGACGGCGACACAGTGTAT
AACAGCGCCCTGAAATCTAGGCTGTCCATCAGCCGGGACACTTCTAAGAACCAGGTGTT
TCTGAAAATGAATTCCCTGGAGACTGATGAAACAGGCACTTACTATTGCACCAGGGACT
TCGGGTACTTTGATGGTTCCAGCCCATTCGACTATTGGGGCCAGGGAGTGATGGTCAC
CGTGTCTAGTGCTAGTACAAAGGGGCCCAGCGTGTTTCCACTGGCACCCTCATCCAAAT
CTACTAGTGGAGGAACCGCCGCTCTGGGCTGTCTGGTGAAGGATTATTTCCCCGAGCC
TGTCACCGTGAGCTGGAATTCTGGCGCACTGACAAGCGGAGTCCACACTTTTCCAGCC
GTGCTGCAGAGCTCTGGCCTGTACTCCCTGAGTTCAGTGGTCACAGTCCCCTCCAGCT
CTCTGGGAACCCAGACATACATCTGTAACGTGAACCATAAGCCTAGCAACACTAAGGTG
GACAAAAAGGTGGAGCCAAAATCCTGCGACAAAACCCATACATAA
SEQ ID NO: 14 - Nucl. Acid of VL Fab6A10 with IgL secretion sequence: 
ATGTCCGTGCCTACCCAGGTGCTGGGGCTGCTGCTGCTGTGGCTGACTGATGCAAGAT
GTCCCGTCGATATTGTGCTGACCCAGAGCCCAGCCACACTGAGTGTGACTCCCGGAGA
GTCTGTCAGTCTGTCATGCAGGGCTAGCCAGGGGATCTCCACCAGCATTCACTGGTAC
CAGCAGAAGAGTAACGAATCACCCAGGCTGCTGATCAAATTCGCCTCTCAGAGTATCTC
AGGAATTCCATCTCGGTTCTCCGGCAGCGGATCTGGGACTGACTTTACCCTGTCCATTA
ATAGAGTGGAGAGCGAAGATTTCTCTGTCTATTTTTGCCAGCAGACATACAGCCTGCCC
TATACCTTCGGTGCAGGCACAAAGCTGGAGCTGAAACGCACTGTGGCCGCTCCTAGCG
TCTTCATCTTTCCCCCTTCTGACGAACAGCTGAAGAGTGGTACAGCTTCAGTGGTCTGT
CTGCTGAACAATTTTTACCCACGAGAGGCAAAGGTGCAGTGGAAAGTCGATAACGCCCT
GCAGTCCGGCAATAGCCAGGAGTCTGTGACTGAACAGGACAGTAAGGATTCAACCTATT
CCCTGTCCAGCACCCTGACACTGTCCAAAGCTGACTACGAGAAGCATAAAGTGTATGCA
TGTGAAGTCACCCACCAGGGGCTGTCATCACCCGTCACAAAATCATTCAATAGGGGGG
AGTGCTAA
SEQ ID NO: 15 - IgG secretion sequence: MYLGLNYVFIVFLLNGVQS
SEQ ID NO: 16 - IgL secretion sequence: MSVPTQVLGLLLLWLTDARC
SEQ ID NO: 17 - N-terminal fragment of the VL of Fab6A10 PVDIVLTQSPATLSV
SEQ ID NO: 18 - N-terminal fragment of the VL of Fab6A10 RCPVDIVLTQSPATLSV
SEQ ID NO: 19 - N-terminal fragment of the VH of Fab6A10 QVQLKESGPGLVQPS
SEQ ID NO: 20 - C-terminal fragment of the VL of Fab6A10 GLSSPVTKSFNRGEC
SEQ ID NO: 21 - C-terminal fragment of the VH of Fab6A10 VDKKVEPKSCDKTHT

The present invention is further illustrated by the following examples. Yet, the examples and specific embodiments described therein must not be construed as limiting the invention to such specific embodiments.

### Examples

### Generation of CHO cell line producing 6A10 Fab fragments

A Chinese hamster ovary (CHO)-derived cell line clone expressing 6A10Fab was established using recombinant DNA technology. Construction of the production cell line was performed by Fraunhofer ITEM, PhB, Braunschweig in responsibility of Helmholtz Zentrum München. The host suspension cell line CHO HIT was developed from CHO K1 (ECACC, No. 85051005).

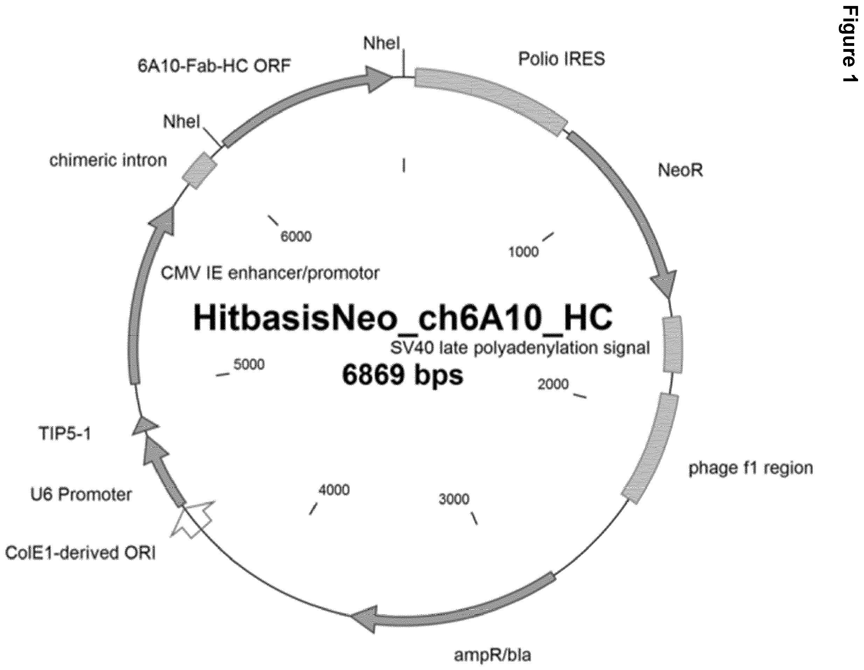

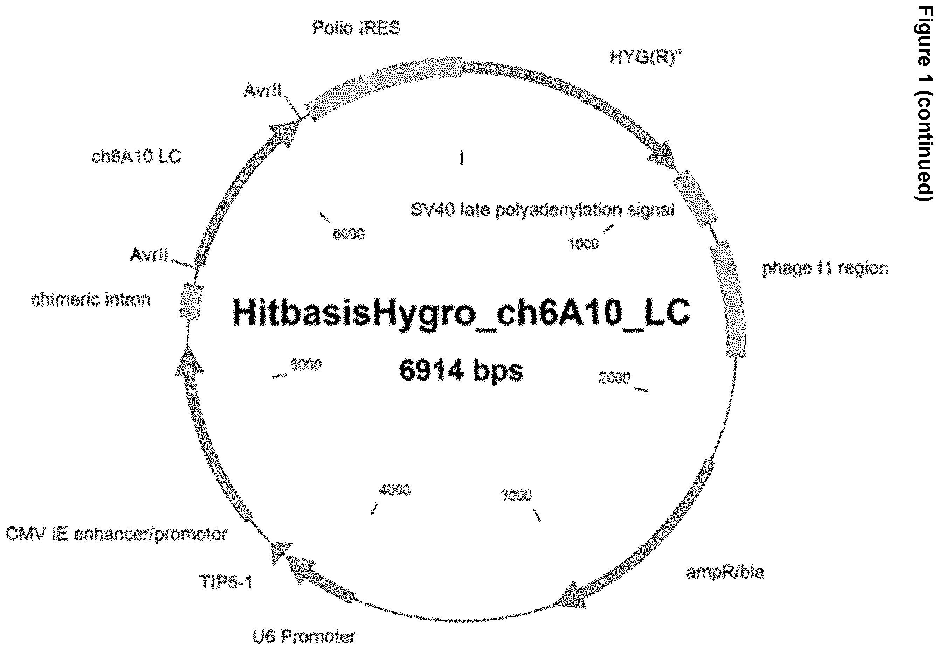

The vectors HitbasisHygro_ch6A10_LC and HitbasisNeo_ch6A10_HC (see Figure 1) were generated for the transfection of CHO HIT cells. For this purpose, the coding sequence of 6A10Fab light chain and heavy chain were amplified using PCR-technology from plasmids pUC57-ch6A10 LC and pUC57-ch6A10 HC. The 6A10Fab light chain cDNA was inserted into HITbasisHygro. The 6A10Fab heavy chain cDNA was inserted into HITbasisNeo.

The corresponding vector structure is provided in Figure 1. The combined results from restriction analysis and partial sequencing proved the correctness and integrity of the plasmid.

After the expression vector was transformed into competent *E.coli* TopTen cells, the cells were plated onto LB agar media containing ampicillin. Resistant colonies were selected to isolate plasmid DNA.

For preparation of host cell line, one vial of the CHO HIT host cell line was thawed, inoculated into "Host cell growth medium" (ProCHO5 medium containing 4 mM GlutaMAX) and was expanded for transfection.

Transfection of both expression vectors (either together or successive) into the host cells was performed by electroporation using AMAXA nucleofection system (LONZA) according to manufacturer's manual. The transfected cells were incubated at 37 °C.

Two days after transfection, the cell pools were transferred into "Selective medium 1" or "Selective medium 2" (proCHO medium containing 4 mM GlutaMAX and antibiotics) and subcultivated until viability recovered and cells started to grow. For pools with acceptable growth, titers were determined by densitometric evaluation of SDS-PAGE and cell specific productivities were estimated. The cell pool "MP P6-A4" was selected for single cell cloning.

Clones were isolated in an image-supported cloning procedure using limiting dilution method combined with image-based documentation. Hereafter all grown clones were expanded for later freezing of 5 vials each. The clones showing the best product concentrations as determined by PAIA assay were expanded up to shake flask level (9 clones). The clones "P1D20", "P1E20", "P1H06" and "P5E17" were chosen as preferential production cell lines and used for an expression stability study. Based on obtained data during expression stability study, the clones "P1E20" and "P5E17" were considered as sufficiently phenotypically stable. Finally, the cell clone "P1E20" was selected for the development of an USP process based on quantity and quality.

### N- and C-terminal sequencing

N- and C-terminal sequencing by MALDI-TOF-MS is a method for verification of the N- and C- terminal amino acid sequence of the protein. The reduced sample is spotted on a ground steel target using 1,5-diaminonapthalene and measured with MALDI-TOF-MS in positive ion mode. Fragmentation along the peptide amino acid backbone leads to fragments specific for the N- and C-terminal amino acid sequence of the protein. The theoretical amino acid sequence is verified by comparing the experimentally determined molecular masses of the fragments to the theoretically expected fragments.

The identity of 6A10Fab-CHX-A"-DTPA was verified by N- and C-terminal sequencing using MALDI-ISD-MS. Sequencing detected additional arginine (R) and cysteine (C) amino acids at the N- terminus of the light chain; cf. Table 2. No such additions or modifications were observed at the heavy chain.

**Table 2: N- and C-termini of 6A10Fab chains (reference and batch)**

| **Subject** | **N-terminus (first AAs)** | **C-terminus (last AAs)** |
|---|---|---|
| 6A10Fab light chain (reference (natural) sequence) | --PVDIVLTQSPATLSV (SEQ ID NO: 17) | GLSSPVTKSFNRGEC (SEQ ID NO: 20) |
| 6A10 Fab light chain (Batch 19AI002) | RCPVDIVLTQSPATLSV (SEQ ID NO: 18) | GLSSPVTKSFNRGEC (SEQ ID NO: 20) |
| 6A10 Fab heavy chain (reference (natural) sequence) | QVQLKESGPGLVQPS (SEQ ID NO: 19) | VDKKVEPKSCDKTHT (SEQ ID NO: 21) |
| 6A10 Fab heavy chain (Batch 19AI002) | QVQLKESGPGLVQPS (SEQ ID NO: 19) | VDKKVEPKSCDKTHT (SEQ ID NO: 21) |

## Claims

1. A dimer composed of a first Fab monomer and a second Fab monomer, each Fab monomer comprising a VH and a VL region, wherein
(i) the VL region of the first Fab monomer and the VL region of the second Fab monomer are covalently linked by a disulfide bond between an additional non-naturally occurring cysteine residue at the N-terminus of the VL region of the first Fab monomer and the N-terminus of the VL region of the second Fab monomer;
(ii) the VH region of the first Fab monomer and the VH region of the second Fab monomer are covalently linked by a disulfide bond between an additional non-naturally occurring cysteine residue at the N-terminus of the VH region of the first Fab monomer and the N-terminus of the VH region of the second Fab monomer;
(iii) the VL region of the first Fab monomer and the VH region of the second Fab monomer are covalently linked by a disulfide bond between an additional non-naturally occurring cysteine residue at the N-terminus of the VL region of the first Fab monomer and the N-terminus of the VH region of the second Fab monomer; or
(iv) the VH region of the first Fab monomer and the VL region of the second Fab monomer are covalently linked by a disulfide bond between an additional non-naturally occurring cysteine residue at the N-terminus of the VH region of the first Fab monomer and the N-terminus of the VL region of the second Fab monomer.

2. The dimer of claim 1, wherein the non-naturally occurring cysteine residue at the N-terminus of the VL region or VH region is at amino acid position 1 or amino acid position 2, wherein amino acids are counted from the N-terminus of the VL region or VH region, respectively, with position 1 being the first amino acid at the N-terminus.

3. The dimer of claim 1 or 2, wherein said first Fab monomer and said second Fab monomer are the same or are different.

4. The dimer of any one of the preceding claims, wherein said first Fab monomer and said second Fab monomer are directed against the same target.

5. The dimer of any one of claims 1 to 3, wherein said first Fab monomer is directed against a first target and said second Fab monomer is directed against a second target, said first and second target being different.

6. The dimer of any one of the preceding claims, wherein said first Fab monomer comprises the VH-CDR1 shown in SEQ ID NO: 1, the VH-CDR2 shown in SEQ ID NO: 2, the VH-CDR3 shown in SEQ ID NO: 3, the VL-CDR1 shown in SEQ ID NO: 4, the VL-CDR2 shown in SEQ ID NO: 5 and the VL-CDR3 shown in SEQ ID NO: 6.

7. The dimer of any one of the preceding claims, wherein the VL region of said first Fab monomer and the VL region of said second Fab monomer comprises at its N-terminus the secretion sequence shown in SEQ ID NO: 16.

8. The dimer of any one of the preceding claims, wherein the VH region of said first Fab monomer and the VH region of said second Fab monomer comprises at its N-terminus the secretion sequence shown in SEQ ID NO: 16.

9. The dimer of any one of the preceding claims, wherein the VL region of said first Fab monomer and the VH region of said second Fab monomer comprises at its N-terminus the secretion sequence shown in SEQ ID NO: 16.

10. The dimer of any one of the preceding claims, wherein the VH region of said first Fab monomer and the VL region of said second Fab monomer comprises at its N-terminus the secretion sequence shown in SEQ ID NO: 16.

11. The dimer of any one of the preceding claims, wherein said first Fab monomer comprises the VH region shown in SEQ ID NO: 7 and the VL region shown in SEQ ID NO: 8.

12. The dimer of any one of the preceding claims, wherein said first Fab monomer comprises the VH region shown in SEQ ID NO: 11 and the VL region shown in SEQ ID NO: 12.

13. The dimer of any one of the preceding claims, which is obtainable by
(a) culturing a CHO cell expressing a first and a second Fab monomer as defined in any one of claims 1 to 12 until stationary phase is reached;
(b) optionally adding an oxidant to the CHO cell culture; and
(c) harvesting said dimer.

14. A composition comprising the dimer of any one of claims 1 to 13.

15. The composition of clam 14, further comprising Fab monomers as defined in any one of claims 1 to 13.
